(19) Europäisches Patentamt
European Patent Office
Office européen des brevets

(11) Numéro de publication: **0 126 012**
**B1**

(12) # FASCICULE DE BREVET EUROPÉEN

(45) Date de publication du fascicule du brevet: **13.01.88**

(51) Int. Cl.⁴: **A 61 K 31/155,** A 61 K 31/395, A 61 K 31/16

(21) Numéro de dépôt: **84401006.6**

(22) Date de dépôt: **17.05.84**

(54) **Compositions pharmaceutiques à base de disulfures symétriques.**

(30) Priorité: **17.05.83 FR 8308153**
**17.05.83 FR 8308154**

(43) Date de publication de la demande:
**21.11.84 Bulletin 84/47**

(45) Mention de la délivrance du brevet:
**13.01.88 Bulletin 88/2**

(84) Etats contractants désignés:
**BE CH DE FR GB IT LI LU NL SE**

(56) Documents cité:
**EP-A-0 006 227**
**EP-A-0 010 361**
**DE-C-960 279**
**FR-A-1 006 624**
**GB-A-831 075**

(73) Titulaire: **SANOFI, société anonyme, 40, Avenue George V, F-75008 Paris (FR)**
Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Beuzard, Yves, 7, rue de Villersexel, F-75007 Paris (FR)**
Inventeur: **Garel, Marie- Claude, 22, route d'Hericy, F-77870 Vulaines- sur- Seine (FR)**
Inventeur: **Demarne, Henri, Le Florence Avenue Major Flandre, F-34000 Montpellier (FR)**
Inventeur: **Sebille, Bernard, 45 bis, rue Pierre Louvrier, F-92140 Clamart (FR)**

(74) Mandataire: **Gillard, Marie- Louise, Cabinet Beau de Loménie 55, Rue d'Amsterdam, F-75008 Paris (FR)**

**EP 0 126 012 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

Il est connu que la drépanocytose est une maladie génétique qui comporte une anomalie de la structure de l'hémoglobine dont l'acide aminé Glu-6 de la chaîne bêta est remplacé par l'acide aminé Val pour donner l'hémoglobine S qui polymérise.

Ladite polymérisation lors de la désoxygénation du globule rouge, entraîne la falciformation de ce dernier qui devient rigide, circule mal et se bloque dans les petits vaisseaux. Les individus porteurs de deux gènes drépanocytaires sont ainsi sous la menace permanente d'une complication fatale.

Certains disulfures ont été décrits dans la littérature comme inhibant la falciformation; notamment la cystamine a été indiquée comme particulièrement active ("Developments of Therapeutic Agents for Sikle Cell Disease", Inserm Symposium 1979, Horth Holland: Amsterdam Editeurs J. Rosa, Y. Beuzard, J. Hercules; p. 139-153).

Il a été maintenant trouvé que les disulfures de formule I:

R - S - S - R (I)

dans laquelle R représente:

- un groupe

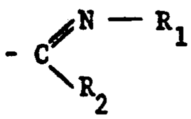

dans lequel $R_1$ représente l'hydrogène et $R_2$ représente un groupe $NH_2$ ou encore $R_1$ et $R_2$ pris avec les atomes d'azote et de carbone auxquels ils sont attachés forment un radical 4,5-dihydro-1H-imidazol-2-yle, 4,5-dihydro-1-méthyl-1H-imidazol-2-yle, thiazol-2-yle, 4,5-dihydro-thiazol-2-yle, 1H-1,2,4-triazol-3-yle, 1-méthyl-1H-1,2,4-triazol-3-yle, pyrimidin-2-yle ou quinoléin-2-yle;

- un groupe

- un groupe 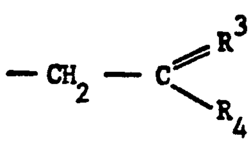

dans lequel $R_3$ représente un atome d'oxygène ou un groups NH et $R_4$ représente un groupe

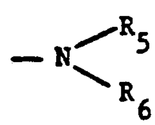

où $R_5$ et $R_6$ représentent indépendamment l'hydrogène, méthyle ou éthyle ou bien $R_3$ et $R_4$, ensemble représentent un atome d'azote;

ainsi que leurs sels éventuels, sont très actifs dans l'inhibition de la polymérisation de l'hémoglobine S et de la falciformation des globules rouges.

Il a été également trouvé que les disulfures symétriques de formule I ci-dessus sont actifs sur les parasites affectant les érythrocytes, tels que les Plasmodia et les Babesiae. Plus particulièrement, ils montrent une activité schizonticide in vitro sur "Plasmodium falciparum" et in vivo sur "Plasmodium berghei".

Par conséquent, l'expression "inhibition de la malformation ou de la destruction des érythrocytes", telle qu'utilisée ici, définit une inhibition qui peut être soit directe sur l'hémoglobine soit indirecte par l'inhibition de la croissance des parasites dans le globule rouge.

L'inhibition de la falciformation des érythrocytes humains a été évaluée selon la méthode consistant à laver et incuber dans un tampon salin (pH 7,40 - 0,15 M), les globules rouges des drépanocytaires pendant une heure à 37°C dans un bainmarie sous agitation circulaire en présence du produit ci-dessus à différentes concentrations. Dans cette opération le rapport molaire produit/hémoglobine est testé initialement dans l'intervalle allant de 0,5 à 20. En fonction des résultats obtenus, ce rapport est modifié. A la fin de l'incubation, l'excès de produit est enlevé par lavage. La suspension cellulaire ajustée à un hématocrite de 5 % est transférée dans un Erlenmeyer et incubée à 37°C sous un courant d'un mélange humidifié d'azote et d'oxygène. La concentration d'oxygène est réglée par une pompe à mélange gazeux. Le gaz effluent est transféré dans un autre tube contenant du formaldéhyde. A la fin de l'incubation, la suspension cellulaire est transférée dans le formaldéhyde par simple retournement de la fiole.

La proportion des cellules déformées et de celles ayant les caractéristiques de drépanocytes (présentant deux prolongements filiformes) est évaluée à l'aide d'un microscope ayant une optique interférentielle de NOMARSKY. L'inhibition de la falciformation a été calculée selon la formule suivante:

% cellules falciformées témoins - % cellules falciformées en présence de la drogue

% cellules falciformées du témoin

Le tableau I montre le pourcentage d'inhibition de la falciformation des érythrocytes pour certains composés inclus dans la formule I ci-dessus en comparaison avec la cystamine.

**Tableau I**

| Composé | % inhibition |
|---|---|
| cystamine | 33 |
| dithiodiformamidine | 100 |
| 2,2'-dithiodi(1H-imidazole) | 80 |
| 2,2'-dithiodi(4,5-dihydrothiazole) | 90 |
| 3,3'-dithiodi(1H-1,2,4-triazole) | 100 |
| 2,2'-dithiodipyrimidine | 50 |
| dithio-diacétamide | 80 |
| dithiodi(N,N-diméthyl)acétamide | 95 |

De ce tableau il ressort que les composés qui sont inclus dams la formule I ci-dessus inhibent la falciformation des érythrocytes des drépanocytaires et sont beaucoup plus actifs que le composé de référence.

Ainsi, la présente invention se réfère à des compositions pharmaceutipues pour l'inhibition de la malformation ou de la destruction des érythrocytes due à une modification génétique de l'hémoglobine ou à des parasites renfermant, en tant qu'ingrédient actif, un disulfure de formule:

R -- S -- S -- R

dans lequel R représente:

un groupe C

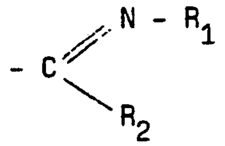

dans lequel $R_1$ représente l'hydrogène et $R_2$ représente un groupe $NH_2$ ou encore $R_1$ et $R_2$ pris avec les atomes d'azote et de carbone auxquels ils sont attachés forment un radical
4,5-dihydro-1H-imidazol-2-yle,
4,5-dihydro-1-méthyl-1H-imidazol-2-yle,
thiazol-2-yle, 4,5-dihydro-thiazol-2-yle, 1H-1,2,4-triazol-3-yle, 1-méthyl-1H-1,2,4-triazol-3-yle, pyrimidin-2-yle ou quinoléin-2-yle

- un groupe

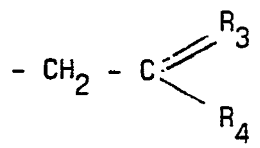

dans lequel $R_3$ représente un atome d'oxygène ou un groupe NH et $R_4$ représente un groupe

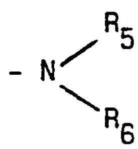

où $R_5$ et $R_6$ représentent indépendamment l'hydrogène, méthyle ou éthyle ou bien $R_3$ et $R_4$, ensemble, représentent un atome d'azote ou un sel éventuel pharmaceutiquement acceptable dudit disulfure.

L'invention a également pour objet l'utilisation des disulfures de formule:

R -- S - S - R

dans laquelle R représente:

un groupe

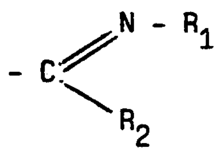

dans lequel $R_1$ et $R_2$ pris avec les atomes d'azote et de carbone auxquels ils sont attachés forment un radical 1H-imidazol-2-yle ou 1-méthyl-1H-imidazol-2-yle, éventuellement sous forme de sel pharmaceutiquement acceptable, pour l'obtention d'un médicament à action inhibant la malformation ou la destruction d'érythrocytes due à une modification génétique de l'hémoglobine ou à des parasites, utile notamment pour le traitement de la drépanocytose, du paludisme et de la babébiose.

Dans les compositions pharmaceutiques de la présente invention, pour l'administration orale, sublinguale, sous-cutanée, intramusculaire, intraveineuse, transdermique ou rectale, les ingrédients actifs de formule I ci-dessus peuvent être administrés sous forme unitaire d'administration, en mélange avec des supports pharmaceutiques classiques, aux animaux et aux êtres humains pour le traitement de la drépanocytose, du paludisme ou de la babébiose. Parmi les formes unitaires d'administration appropriées, il y a les formes par voie orale, telles que les comprimés, les gélules, les poudres les granules et les solutions ou suspensions orales et les formes d'administration sublinguale et buccale, de même que les formes d'administration sous-cutanée, intramusculaire ou intraveineuse, éventuellement par perfusion et les formes d'administration rectale.

Afin d'obtenir l'effet désiré, la dose de principe actif peut varier entre 0,1 et 100 mg par kg de poids du corps et par jour.

Chaque dose unitaire peut contenir de 10 à 1000 mg d'ingrédient actif en combinaison avec un support pharmaceutique. Cette dose unitaire peut être administrée 1 à 4 fois par jour, éventuellement par perfusion, pour le traitement par exemple de la drépanoctyose, du paludisme et de la babébiose.

On prépare simplement les poudres pour l'administration orale en broyant le composé actif à une finesse convenable et en mélangeant avec un diluant broyé similairement. Le diluant peut être un matériau de glucide comestible, tel que l'amidon. Il est avantageux qu'un agent édulcorant ou un sucre ainsi qu'une huile aromatisante soient présents.

On prépare les granules destinés à la reconstitution d'une préparation liquide orale en utilisant des diluants solubles dans l'eau, on mouille le composé actif et un diluant soluble dans l'eau, tel que le saccharose ou le glucose, avec un liant tel que le mucilage d'acacia, une solution de gélatine, une solution de méthylcellulose et on fait passer sur un tamis en forçant pour former des granulés que l'on fait sécher. Il est avantageux d'introduire dans la composition un agent de mise en suspension comme la gomme adragante.

On fait les gélules en préparant un mélange pulvérulent comme il est décrit ci-dessus et en le mettant dans des gaines de gélatine mises en forme. Comme adjuvant à l'opération de remplissage il est avantageux d'ajouter un lubrifiant tel que le talc, le stéarate de magnésium et le stéarate de calcium au mélange pulvérulent avant l'opération de remplissage.

On fait les comprimés en préparant un mélange pulvérulent, en granulant ou en tronçonnant, en ajoutant un lubrifiant et en comprimant pour former des comprimés. On prépare le mélange pulvérulent en mélangeant le composé désiré, convenablement broyé, avec un diluant ou une base, tel que l'amidon, le saccharose, le kaolin, le phosphate bicalcique, etc. On peut granuler le mélange pulvérulent en mouillant avec le liant tel qu'un sirop, une pâte d'amidon ou un mucilage d'acacia et en forçant à travers un tamis. Comme méthode autre que la granulation on peut tronçonner le mélange pulvérulent c'est-à-dire, le faire passer à travers la machine à comprimés et casser les comprimés résultants imparfaitement formés en fragments (tronçons).

On peut lubrifier les tronçons pour les empêcher de coller aux comprimés en formant des cubes, en ajoutant un sel stéarate, du talc ou une huile minérale. On comprime ensuite le mélange lubrifiant pour constituer des comprimés.

Les comprimés peuvent être avantageusement munis d'un revêtement protecteur constitué d'un enduit de scellement de gomme-laque, un revêtement de sucre et de méthylcellulose, et un enduit vernis de cire de carnauba, ou encore on peut les traiter d'une autre manière de telle sorte qu'ils aient une activité prolongée ou retardée et qu'ils libèrent continuellement une quantité prédéterminée de principe actif.

On prépare les fluides pour administration orale sous des formes posologiques unitaires, tellés que sirops où chaque cuillère à café de composition contient une quantité prédéterminée du composé actif à administrer.

Une préparation sous forme de sirop ou d'élixir peut contenir l'ingrédient actif conjointement avec un édulcorant, du Méthylparaben et du Propylparaben comme antiseptiques, ainsi qu'un agent donnant du goût et un colorant approprié.

Pour une application rectale, on recourt à des suppositoires qui sont préparés avec des liants fondant à la température rectale, par exemple du beurre de cacao ou des polyéthylèneglycols.

Pour une application parentérale, on utilise des suspensions aqueuses, des solutions salines isotoniques ou des solutions stériles et injectables qui contiennent des agents de dispersion et/ou mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les composés de formule I ci-dessus sont connus en littérature ou ils peuvent être aisément préparés par oxydation des thiols correspondants selon des méthodes de synthèse bien connues (Houben-Weyl, Methoden der Organischen Chemie, Vol. IX, p. 19, 4e Ed., G. Thieme-Verlag Stuttgart).

Les exemples suivants illustrent l'invention sans toutefois la limiter.

**Préparations**

Le composé (I) où R représente

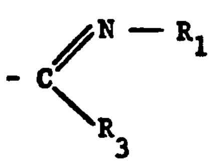

avec $R_1 = H$ et $R_2 = -NH_2$ (dithiodiformamidine) est un produit bien connu.

Les autres disulfures (I) où R a la signification mentionnée ci-dessus sont aisément préparés par oxydation des thiols correspondants par l'iode, par le brome ou à l'air.

A titre d'exemple, on indiquera ci-après la préparation du 2,2'-dithio-diimidazole.

Dans un ballon muni d'un réfrigérant, on place du mercaptoimidazole 1 g, dans un mélange de dichlorométhane (10 ml) et de bicarbonate de sodium à 5 % (20 ml). Le ballon est immergé dans un bain d'eau et une solution de brome (0,26 ml) est ajoutée très lentement au mélange bien agité. Au couts de l'addition la coloration jaune du brome disparait. La solution est laissée encore une heure sous agitation après que tout le brome a été ajouté. La solution est filtrée et un produit insoluble est récupéré. On obtient ainsi 230 mg de 2,2'-dithiodiimidazole. Rendement: 23,2 %; F:158° C.

Les disulfures (I) où R représente 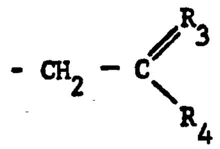

peuvent être obtenus à partir des dérivés halogénés correspondants par action de sulfure de sodium et oxydation simultanée.

A titre d'exemple, on décrit la préparation du dithiodi(N,N-diméthyl)acétamide.

On chauffe à 50° C pendant 2 heures un mélange de 0,24 mol de $Na_2S \cdot 9H_2O$, 0,24 mol de soufre et 200 ml d'eau. Au mélange orange ainsi obtenu, on ajoute, à la température ambiante 0,48 mol de N,N-diméthyl-chloroacétamide. La température monte jusqu'à 40° C et le mélange devient jaune pâle. On chauffe 2 heures à 60° C pour achever la réaction, puis on filtre la solution on évapore l'eau sous pression réduite, on reprend le liquide visqueux ainsi obtenu par de l'acétone et l'on élimine d'abord le chlorure de sodium par filtration et ensuite l'acétone par évaporation sous pression réduite. On obtient ainsi le dithiodi(N,N-diméthyl)- acétamide pur, sous forme d'un liquide très visqueux.

De la même manière on obtient le dithiodiacétonitrile, également sous forme de liquide visqueux et le dithiodiacétamide sous forme de cristaux blancs très fins recristallisés de l'éthanol; F 164° C.

**Exemple 1**

Comprimés ayant la composition suivante:

| | |
|---|---|
| 2,2'-dithiodi(1H-imidazole) | 500 mg |
| glycine | 120 mg |
| cellulose microgranulaire | 70 mg |
| silice précipitée | 18 mg |
| carboxyméthylamidon | 30 mg |
| stéarate de magnésium | 11 mg |
| talc | 11 mg |

On mélange les quantités calculées des composants pendant 30 minutes, puis on granule à sec et on fait passer le mélange à travers un tamis à mailles de 1,6 mm. On comprime ensuite en utilisant un poinçon ayant la forme d'un petit bâton. On obtient ainsi des comprimés pesant chacun 760 mg et renfermant chacun 500 mg de principe actif.

**Exemple 2**

On prépare des comprimés selon l'exemple 1. Les comprimés ainsi obtenus sont enrobés à l'aids d'une suspension de phtalate de dibutyle, de polyméthacrylate de butyle et diméthylaminoéthyle, de polyéthylèneglycol 1500, de silice précipitée, de bioxyde de titanium et de talc dans un mélange acétone: isopropanol 1:1 ayant une concentration en résidu sec de 10 % environ. On obtient ainsi des comprimés enrobés pesant chacun 780 mg et renfermant chacun 500 mg de principe actif.

**Exemple 3**

Comprimés ayant la composition suivante:

| | |
|---|---|
| dithiodiacétamide | 500 mg |
| glycine | 120 mg |
| cellulose microgranulaire | 70 mg |
| silice précipitée | 18 mg |
| carboxyméthylamidon | 30 mg |
| stéarate de magnésium | 11 mg |
| talc | 11 mg |

On opère comme dans l'examplе 1 à partir des constituants mentionnés ci-dessus.

**Example 4**

On prépare un granulé destiné à la reconstitution d'une préparation liquide orale ayant la composition suivante:

| | |
|---|---|
| 2,2'-dithiodi(4,5-dihydrothiazole) | 3,60 g |
| saccharose | 50,00 g |
| carboxyméthylcellulose sodium | 0,80 g |
| acide citrique | 0,10 g |
| citrate trisodique | 0,90 g |
| benzoate de sodium | 0,25 g |
| saccharine sodique | 0,15 g |
| aromatisant | 0,50 g |

Le volume de granulé ainsi obtenu est porté à 100 ml avec de l'eau pour sirops. Une dose unitaire de 5 ml de sirop extemporané ainsi obtenu contient 100 mg de principe actif.

Pour préparer le granulé, on pulvérise les quantités calculées de tous les composants, sauf le saccharose, puis on mélange la poudre ainsi obtenue avec le saccharose jusqu'à obtenir un granulé homogène.

**Exemple 5**

On prépare selon l'exemple 4 un granulé destiné à la reconstitution d'une préparation liquide orale ayant la composition suivante:

| | |
|---|---|
| 3,3'-dithiodi(1H-1,2,4-triazole) | 7,00 g |
| saccharose | 46,60 g |
| carboxyméthylcellulose sodium | 0,90 g |
| acide citrique | 0,10 g |
| citrate trisodique | 0,90 g |
| benzoate de sodium | 0,25 g |
| saccharine sodique | 0,15 g |
| aromatisant | 0,50 g |

Une dose unitaire de 5 ml du sirop extemporané ainsi obtenu contient 350 mg de principe actif.

**Exemple 6**

Selon l'exemple 4, on prépare un granulé destiné à la reconstitution d'une préparation liquide orale ayant la composition suivante:

| | |
|---|---|
| dithiodi(N,N-diméthyl)acétamide | 3,60 g |
| saccharose | 50,00 g |
| carboxyméthylcellulosesodium | 0,80 g |
| acide citrique | 0,10 g |
| citrate trisodique | 0,90 g |
| benzoate de sodium | 0,25 g |
| saccharine sodique | 0,15 g |
| aromatisant | 0,50 g |

**Exemple 7**

On prépare selon l'exemple 4 un granulé destiné à la reconstitution d'une préparation liquide orale ayant la composition suivante:

| | |
|---|---|
| dithiodiacétamide | 7,00 g |
| saccharose | 46,60 g |
| carboxyméthylcellulose sodium | 0,90 g |
| acide citrique | 0,10 g |
| citrate trisodique | 0,90 g |
| benzoate de sodium | 0,25 g |
| saccharine sodique | 0,15 g |
| aromatisant | 0,50 g |

**Exemple 8**

On prépare selon l'exemple 4 un granulé destiné à la reconstitution d'une préparation liquide orale ayant la composition suivante:

| | |
|---|---|
| 2,2'-dithiodipyrimidine | 8,00 g |
| saccharose | 45,60 g |
| carboxyméthylcellulose sodium | 1,00 g |
| acide citrique | 0,10 g |
| citrate trisodique | 0,90 g |
| benzoate de sodium | 0,25 g |
| saccharine sodique | 0,15 g |
| aromatisant | 0,50 g |

Une dose unitaire de 5 ml du sirop extemporané ainsi obtenu contient 400 mg de principe actif.

**Exemple 9**

On prépare selon l'exemple 4 un granulé destiné à la reconstitution d'une préparation liquide orale ayant la composition suivante:

| | |
|---|---|
| dithiodiacétamidine | 8,00 g |
| saccharose | 45,60 g |
| carboxyméthylcellulose sodium | 1,00 g |
| acide citrique | 0,10 g |
| citrate trisodique | 0,90 g |
| benzoate de sodium | 0,25 g |
| saccharine sodique | 0,15 g |
| aromatisant | 0,50 g |

Une dose unitaire de 5 ml du sirop extemporané ainsi obtenu contient 400 mg de principe actif.

**Exemple 10**

On prépare des comprimés ayant la composition suivante:

| | |
|---|---|
| dithiodiacétonitrile | 350 mg |
| cellulose microcristalline | 100 mg |
| lactose | 125 mg |
| stéarate de magnésium | 10 mg |
| talc | 15 mg |

On fait passer les poudres à travers un tamis de 3 mm, puis on mélange les ingrédients jusqu'à ce qu'on obtienne un mélange homogène qui est comprimé et granulé. Les granules ainsi obtenus sont utilisés pour former des comprimés par compression. Poids d'un comprimé: 600 mg.

**Exemple 11**

Selon l'exemple 10, on prépare des comprimés ayant la composition suivante:

| | |
|---|---|
| dithiodiformamidine | 350 mg |
| cellulose microcristalline | 100 mg |
| lactose | 125 mg |
| stéarate de magnésium | 10 mg |
| talc | 15 mg |

On fait passer les poudres à travers un tamis de 0,3 mm, puis on mélange les ingrédients jusqu'à ce qu'on obtienne un mélange homogène qui est comprimé et granulé. Les granules ainsi obtenus sont utilisés pour former des comprimés par compression. Poids d'un comprimé: 600 mg.

**Exemple 12**

Comprimés ayant la composition suivante:

| | |
|---|---|
| 2,2'-dithiodi(1-méthyl-1H-imidazole) | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

On mélange intimement dans une mélangeuse-pétrisseuse tous les ingrédients, sauf le lubrifiant, pendant 15 minutes, puis on pétrit par addition graduelle d'eau. On fait passer la masse à travers un tamis de 1,25 mm. On sèche le granulé dans une étuve à ventilation forcée jusqu'à ce qu'on obtienne une humidité résiduelle relativement réduite (environ 2 %). On uniformise le granulé, on ajoute le lubrifiant et on forme des comprimés par compression. Poids d'un comprimé: 300 mg.

De la même façon on prépare des comprimés contenant 250 mg de principe actif.

**Exemple 13**

Selon l'exemple 12, on prépare des comprimés ayant la composition suivante:

| | |
|---|---|
| dithio(N,N-diéthyl)acétamide | 150 mg |
| cellulose microcristalline | 75 mg |
| talc | 15 mg |
| polyvinylpyrrolidone | 30 mg |
| silice précipitée | 25 mg |
| stéarate de magnésium | 5 mg |

**Exemple 14**

On prépare des comprimés ayant la composition suivante:

| | |
|---|---|
| 2,2'-dithiodi-quinoléine | 150 mg |
| carboxyméthylamidon | 10 mg |
| cellulose microcristalline | 85 mg |
| lactose | 135 mg |
| huile de ricin hydrogénée | 10 mg |
| stéarate de magnésium | 5 mg |

en opérant comme décrit à l'exemple 10. On recouvre les comprimés ainsi obtenus par un revêtement ayant la composition suivante:

| | |
|---|---|
| phtalate de butyle | 0,300 mg |
| polyméthacrylate de butyle de diméthylaminoéthyle | 1,850 mg |
| polyéthylèneglycol 1500 | 0,080 mg |
| silice précipitée | 0,020 mg |
| talc | 0,900 mg |
| bioxyde de titane | 1,850 mg |

dissous dans un solvant qui est éliminé par évaporation dans une étuve à ventilation forcée. Poids d'un comprimé: 400 mg

**Exemple 15**

Comme dans l'exemple 14 on prépare des comprimés ayant la composition suivante:

| | |
|---|---|
| dithiodi(N,N-diméthyl)acétamide | 150 mg |
| carboxyméthylamidon | 10 mg |
| cellulose microcristalline | 85 mg |
| lactose | 135 mg |
| huile de ricin hydrogénée | 10 mg |
| stéarate de magnésium | 5 mg |

On recouvre les comprimés ainsi obtenus par un revêtement ayant la composition suivante:

| | |
|---|---|
| phtalate de butyle | 0,300 mg |
| polyméthacrylate de butyle de diméthylaminoéthyle | 1,850 mg |
| polyéthylèneglycol 1500 | 0,080 mg |
| silice précipitée | 0,020 mg |
| talc | 0,900 mg |
| bioxyde de titane | 1,850 mg |

**Exemple 16**

Suppositoires ayant la composition suivante:

| | |
|---|---|
| dithiodiacétamide | 300 mg |
| masse pour suppositoires | 1,450 mg |

On met en suspension la substance active finement pulvérisée dans la masse pour suppositoires à 37° C et on verse le mélange dans des moules légèrement refroidis au préalable. Poids d'un suppositoire: 1 750 mg.

**Exemple 17**

En opérant comme dans l'exemple 16, on prépare des suppositoires ayant la composition suivante:

| | |
|---|---|
| 2,2'-dithiodi(1H-imidazole) | 300 mg |
| masse pour suppositoires | 1 450 mg |

**Revendications**

1. Composition pharmaceutique pour l'inhibition de la malformation ou de la destruction des érythrocytes due à une modification génétique de l'hémoglobine ou à des parasites, utile notamment pour le traitement de la drépanocytose, du paludisme et de la babébiose, caractérisée en ce qu'elle contient en tant que principe actif, un disulfure symétrique de formule:
R -- S -- S -- R

dans lequel R représente:
- un groupe

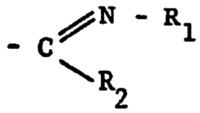

dans lequel $R_1$ représente l'hydrogène et $R_2$ représente un groupe $NH_2$ ou encore $R_1$ et $R_2$ pris avec les atomes d'azote et de carbone auxquels ils sont attachés forment un radical 4,5-dihydro-1H-imidazol-2-yle, 4,5-dihydro-1-méthyl-1H-imidazol-2-yle, thiazol-2-yle, 4,5-dihydro-thiazol-2-yle, 1H-1,2,4-triazol-3-yle, 1-méthyl-1H-1 2,4-triazol-3-yle, pyrimidin-2-yle ou quinoléin-2-yle;

- un groupe

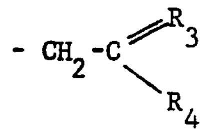

dans lequel $R_3$ représente un atome d'oxygène ou un groupe NH et $R_4$ représente un groupe

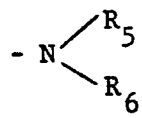

où $R_5$ et $R_6$ représentent indépendamment l'hydrogène, méthyle ou éthyle ou bien $R_3$ et $R_4$, ensemble, représentent un atome d'azote; éventuellement sous forme de sel pharmaceutiquement acceptable.

2. Composition selon la revendication 1, caractérisée en ce qu'elle est sous forme d'unité de dosage.

3. Composition selon la revendication 2 caractérisée en ce que chaque unité de dosage contient de 10 à 1 000 mg de principe actif.

4. Utilisation d'un disulfure symétrique de formule:
R -- S -- S -- R
dans laquelle R représente:

Un groupe

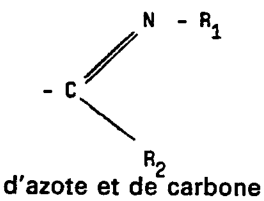

dans lequel $R_1$ et $R_2$ pris avec les atomes d'azote et de carbone auxquels ils sont attachés forment un radical 1H-imidazol-2-yle ou 1-méthyl-1H-imidazol-2-yle, éventuellement sous forme de sel pharmaceutiquement acceptable, pour l'obtention d'un médicament à action inhibant la malformation ou la destruction des érythrocytes dues à une modification génétique de l'hémoglobine ou à des parasites, utile notamment pour le traitement de la drépanocytose, du paludisme et de la babébiose.

5. Utilisation selon la revendication 4, caractérisée en ce que le médicament est sous forme d'unité de dosage.

6. Utilisation selon la revendication 5, caractérisée en ce que chaque unité de dosage contient de 10 à 1000 mg de disulfure symétrique.

## Patentansprüche

1. Pharmazeutische Zusammensetzung zur Hemmung der auf einer genetischen Modifikation des Hämoglobins oder auf Parasiten beruhenden Mißbildung oder Zerstörung der Erythrocyten und zur Behandlung von insbesondere der Drepanocytose, des Paludismus und der Babesiose, gekennzeichnet durch den Gehalt eines symmetrischen Disulfids der Formel
R - S - S - R

als Wirkstoff,

in der R bedeutet:

- eine Gruppe

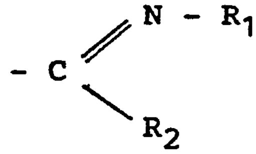

in der $R_1$ Wasserstoff und $R_2$ $NH_2$ sind oder $R_1$ und $R_2$ zusammen mit den Stickstoff- und Kohlenstoffatomen, an die sie gebunden sind einen 4,5-Dihydro-1H-imidazol-2-yl-, 4,5-Dihydro-1-methyl-1H-imidazol-2-yl-, Thiazol-2-yl-, 4,5-Dihydro-thiazol-2-yl-, 1H-1,2,4-triazol-3-yl-, 1-Methyl-1H-1,2,4-triazol-3-yl-, Pyrimidin-2-yl- oder Chinolin -2-yl-Rest bilden,

- eine Gruppe

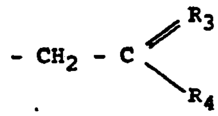

in der $R_3$ Sauerstoff oder NH und $R_4$ eine Gruppe

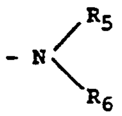 sind,

sind, wobei $R_5$ und $R_6$ unabhängig Wasserstoff, Methyl oder Ethyl sind, oder $R_3$ und $R_4$ zusammen ein Stickstoffatom darstellen, ggf. in Form eines pharmazeutisch verträglichen Salzes.

2. Zusammensetzung nach Anspruch 1 in Form einer Einzeldosis.

3. Zusammensetzung nach Anspruch 2, dadurch gekennzeichnet, daß jede Einzeldosis von 10 bis 1000 mg Wirkstoff enthält.

4. Verwendung eines symmetrischen Disulfids der Formel

R - S - S - R

in der R

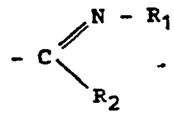

bedeutet, wobei $R_1$ und $R_2$ zusammen mit den Stickstoff- und Kohlenstoffatomen, an die sie gebunden sind, einen 1H-Imidazol-2-yl- oder 1-Methyl-1H-imidazol-2-yl-Rest bilden, ggf. in Form eines pharmazeutisch verträglichen Salzes zur Herstellung eines Medikaments zur Hemmung der auf einer genetischen Modifikation des Hämoglobins oder auf Parasiten beruhenden Mißbildung oder Zerstörung der Erythrocyten und zur Behandlung insbesondere der Drepanocytose, des Paludismus und der Babesiose.

5. Verwendung nach Anspruch 4, dadurch gekennzeichnet, daß das Medikament in Form von Einzeldosen vorliegt.

6. Verwendung nach Anspruch 5, dadurch gekennzeichnet, dan jede Einzeldosis von 10 bis 1000 mg des symmetrischen Disulfids enthält.

## Claims

1. A pharmaceutical composition for the inhibition of the malformation or destruction of the red blood corpuscles due to a genetic modification of the hameoglobin or to parasites, useful in particular in the treatment of drepanocytosis, of malaria and of babebiosis, characterized in that it contains, as active ingredient, a symmetrical disulfide of formula:

R - S - S - R

in which R represents:

a group -

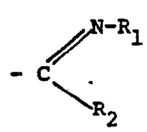

in which $R_1$ represents hydrogen and $R_2$ represents an $NH_2$ group or $R_1$ and $R_2$ taken with the atoms of nitrogen and of carbon to which they are attached form a 4,5-dihydro-1H-imidazol-2-yl, 4,5-dihydro-1-methyl-1H-imidazol-2-yl, thiazol-2-yl, 4,5-dihydrothiazol-2-yl, 1H-1,2,4-triazol-3-yl, 1-methyl-1H-1,2,4-triazol-3-yl, pyrimidin-2-yl or quinolein-2-yl radical;

a group

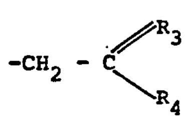

in which $R_3$ represents an atom of oxygen or an NH group and $R_4$ represents

a group -

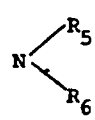

where $R_5$ and $R_6$ independently represent hydrogen, methyl or ethyl or $R_3$ and $R_4$, together, represent an atom of nitrogen; optionally in the form of a pharmaceutically acceptable salt thereof.

2. The composition according to claim 1, characterized in that it is in the form of a unit dosage.

3. The composition according to claim 2, characterized in that each unit dosage contains from 10 to 1000 mg of active ingredient.

4. Use of a symmetrical disulphide of formula:

R - S - S - R

in which R represents:

a group

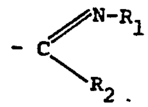

in which $R_1$ and $R_2$ taken with the atoms of nitrogen and of carbon to which they are attached form a 1H-imidazol-2-yl or 1-methyl-1H-imidazol-2-yl, optionally in the form of a pharmaceutically acceptable salt, for obtaining a drug having an action inhibiting the malformation or the destruction of the red blood corpuscles due to a genetic modification of the hameoglobin or to parasites, for use in particular in the treatment of drepanocytosis, of malaria and of babebiosis.

5. Use according to claim 4, characterized in that the drug is in the form of a unit dosage.

6. Use according to claim 5, characterized in that each unit dosage contains from 10 to 1000 mg of symmetrical disulphide.